# EUROPEAN PATENT APPLICATION

(11) **EP 1 829 870 A1**
(43) Date of publication of application: **05.09.2007**
(21) Application number: 05820319.1
(22) Date of filing: 20.12.2005
(51) Int. Cl.: C07D 401/04, C07H 17/08

(54) **2-(PYRAZOL-1-YL)PYRIDINE DERIVATIVE**

(30) Priority: 22.12.2004 JP 2004370710
(71) Applicant: SHIONOGI & CO., LTD., Osaka-shi, Osaka 541-0045 (JP)
(72) Inventor: SHIMIZU, Sumio, Amagasaki-shi, Hyogo 660-0813 (JP); HAKOGI, Toshikazu, Amagasaki-shi, Hyogo 660-0813 (JP)
(74) Representative: Vossius & Partner
(86) International application number: PCT/JP2005/023302
(87) International publication number: WO 2006/068102

(57) **Abstract**

Inventors found that a compound shown by formula: 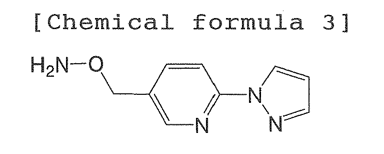 can be produced efficiently by using a compound shown by formula: 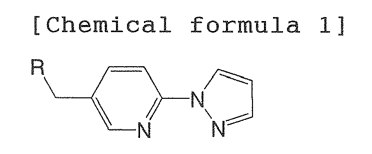 (wherein R is a protected hydroxy or a group shown by: 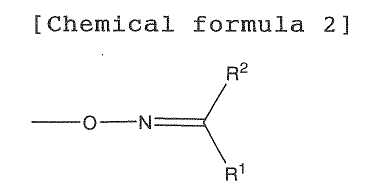 (wherein R¹ is alkyl or optionally substituted aralkyl, and R² is alkyl)).

## Description

### [Technical field]

The present invention relates to 2-(pyrazole-1-yl)pyridine derivative, a method of producing the same, and a method of using the same.

### [Background art]

Patent document 1 discloses a compound which is useful as an antimicrobial agent, represented by the formula: 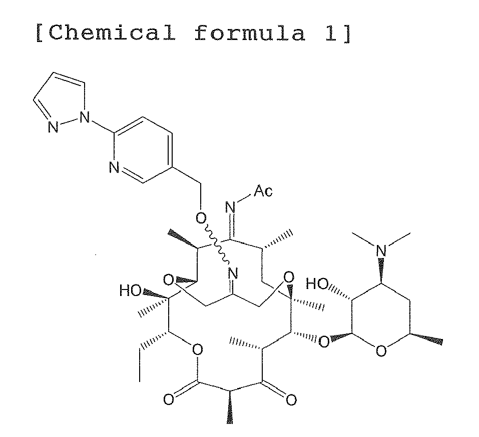 It also discloses as its intermediate, a compound shown by the formula: 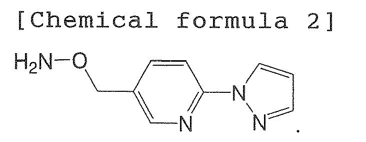 As a production method thereof, the reaction as shown below is described. 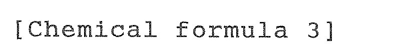 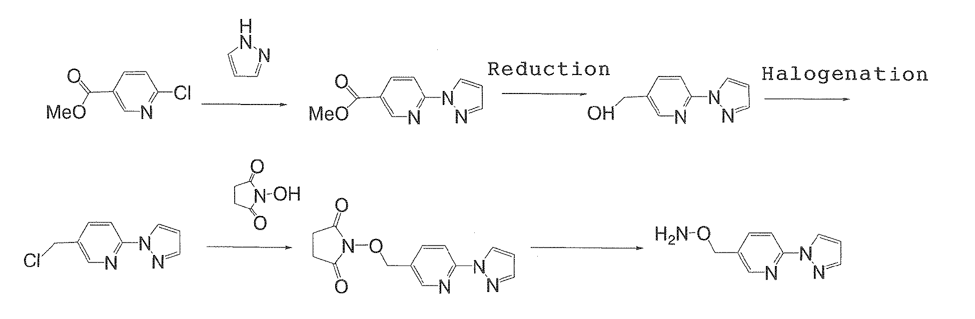 [Patent document 1]
U.S. Patent Publication No. 2004/0171818

### [Disclosure of the invention]

### [Problems to be solved by the invention]

It is an object of the present invention to provide a method of producing an antimicrobial agent shown by formula:

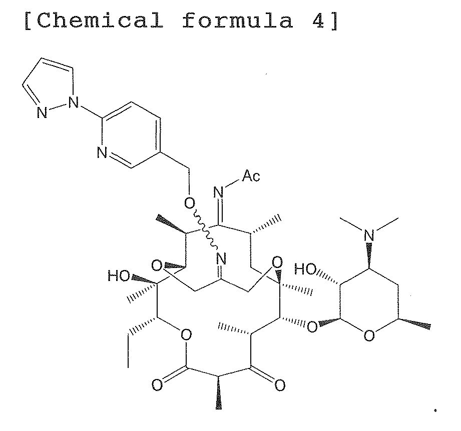

### [Means for solving the problem]

We found a novel synthesis method of a compound shown by formula: 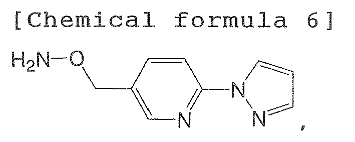 which is an intermediate of an antimicrobial agent shown by: 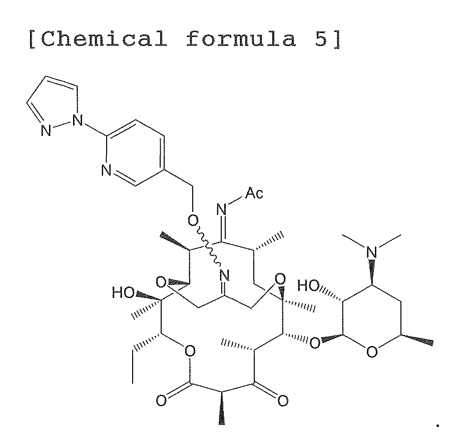 That is, we found a compound shown by the following formula, a method of producing the same and a method of using the same: 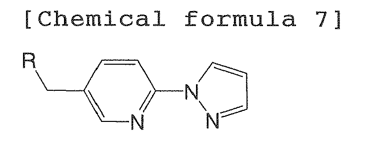 (wherein R is a protected hydroxy or a group shown by formula: 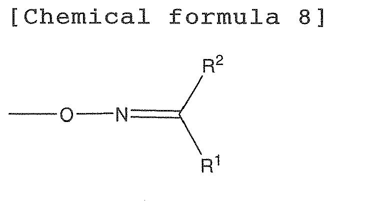 (wherein R¹ and R² each independently represent hydrogen, alkyl, optionally substituted aralkyl or optionally substituted aryl)).
The present invention embraces the following inventions.
(1) A compound shown by formula: 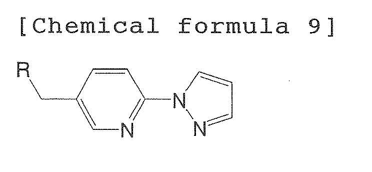 (wherein R is a protected hydroxy or a group shown by formula 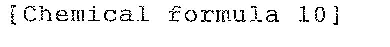 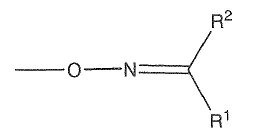 (wherein R¹ and R² each independently represent hydrogen, alkyl, optionally substituted aralkyl, optionally substituted aryl or optionally substituted heteroaryl)).
(2) A method of producing compound shown by formula V 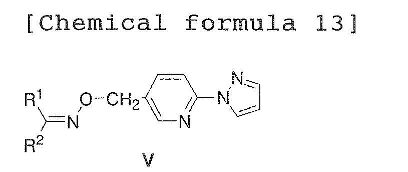 (wherein R¹ and R² each independently represent hydrogen, alkyl, optionally substituted aralkyl, optionally substituted aryl or optionally substituted heteroaryl), comprising reacting a compound shown by formula III: 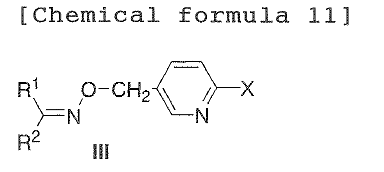 (wherein R¹ and R² are as defined above, X is halogen), with a compound shown by formula IV: 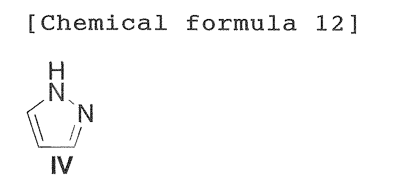 in the presence of base or acid.
(3) A method of producing a compound shown by formula VI or its salt: 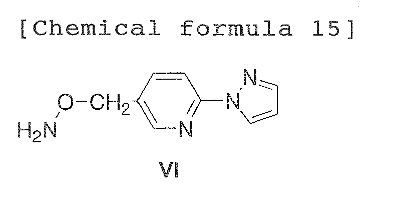 comprising hydrolyzing a compound shown by formula V: 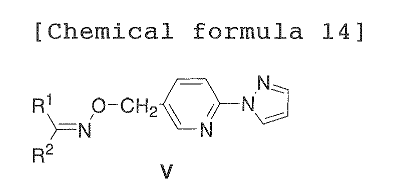 (wherein R¹ and R² each independently represent hydrogen, alkyl optionally substituted aralkyl, optionally substituted aryl or optionally substituted heteroaryl) in the presence of acid.
(4) A method of producing a compound shown by formula IX: 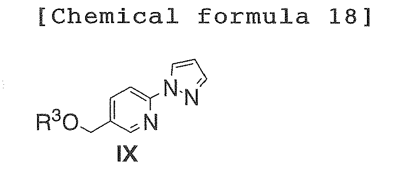 (wherein R³ represents a protective group of hydroxy) comprising reacting a compound shown by formula VIII: 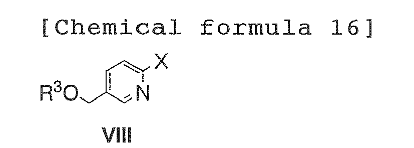 (wherein R³ is as defined above, and X is halogen), with a compound shown by formula IV: 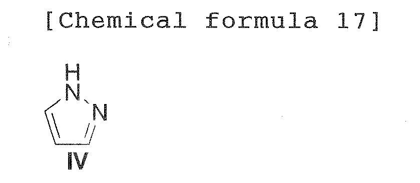 in the presence of base or acid.
(5) A method of producing a compound shown by formula X: 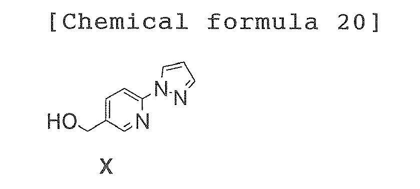 comprising hydrolyzing a compound shown by formula: 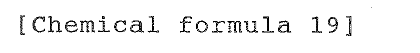 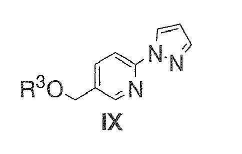 (wherein R³ represents a protective group of hydroxy) in the presence of acid.
(6) A method of producing a compound shown by formula XI: 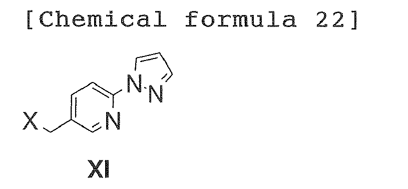 (wherein R is halogen)
   comprising obtaining a compound shown by formula X: 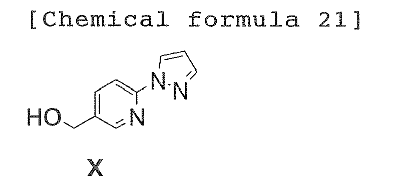 according to the method shown in (5), and reacting the compound shown by formula X with a halogenating agent.
(7) A method of producing a compound shown by formula XIII 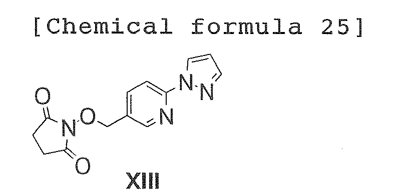 comprising obtaining a compound shown by formula XI: 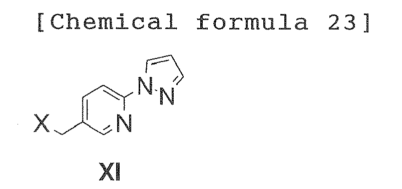 (wherein X is halogen) according to the method shown in (6), and reacting the obtained compound with a compound shown by formula XII: 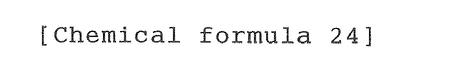 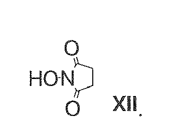
(8) A method of producing a compound or its salt shown by formula VI: 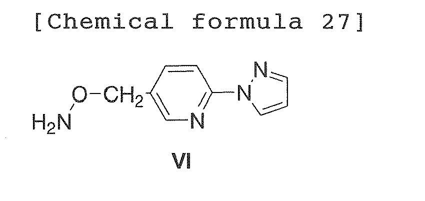 comprising obtaining a compound shown by formula XIII: 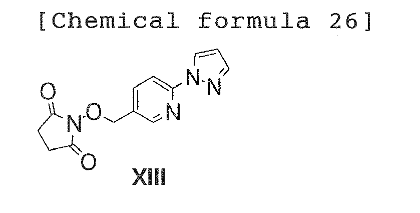 according to the method of (7), and hydrolyzing the obtained compound in the presence of base.
(9) A method of producing a compound shown by formula: 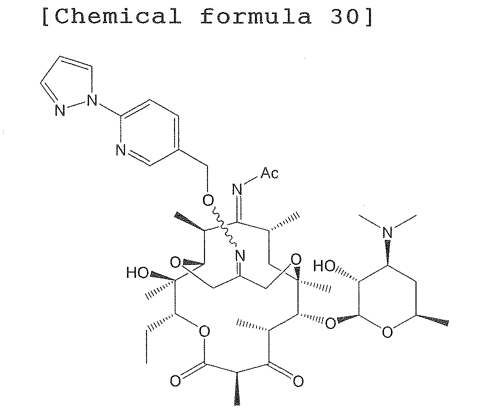 comprising obtaining a compound or its salt shown by formula VI : 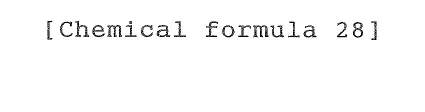 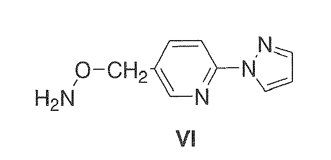 according to the method of (3) or (8), and reacting the obtained compound or its salt with a compound shown by formula: 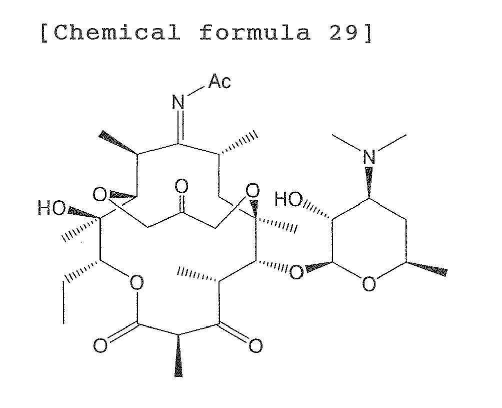 in the presence of acid or base.
(10) A method of producing a compound shown by formula: 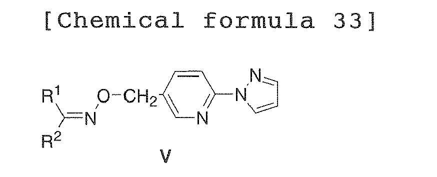 (wherein R¹ and R² each independently represent hydrogen, alkyl, optionally substituted aralkyl or optionally substituted aryl) comprising reacting a compound shown by formula: 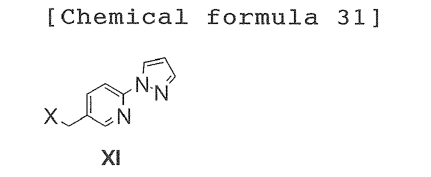 (wherein X is halogen), with a compound shown by formula: 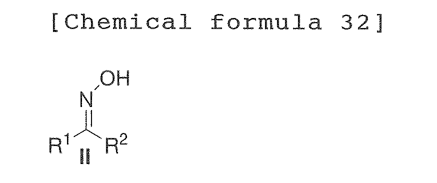 (wherein R¹ and R² are as defined above)).
(11) A salt of a compound shown by formula VI: 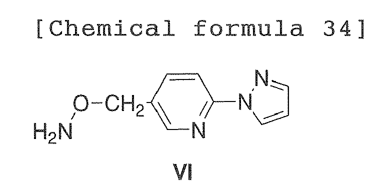
(12) The salt of (11) which is a hydrochloride, sulfate, trichloroacetate or trifluoroacetate.
(13) A compound shown by formula: 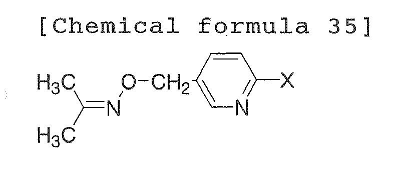 (wherein X is halogen).

### [Best mode for carrying out the invention]

The present compound is a compound shown by formula: 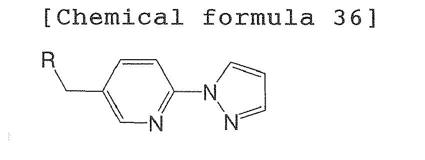 (wherein R is a protected hydroxy or a group shown by formula: 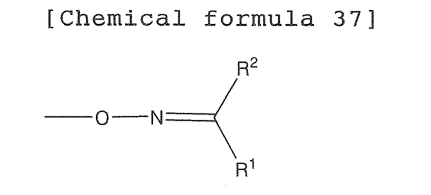 (wherein R¹ and R² each independently represent hydrogen, alkyl, optionally substituted aralkyl, optionally substituted aryl or optionally substituted heteroaryl)).
The term "protected hydroxy" used herein refers to a hydroxy group protected with a protective group. Examples of protective group include tetrahydropyranyl, methoxymethyl, 1-methoxy-1-methylethyl, 1-ethoxyethyl, methyl, benzyl, substituted benzyl and the like. In particular, tetrahydropyranyl is preferred. As a substituted group of substituted benzyl, alkyl, nitro, halogen and the like can be recited.
The term "alkyl" used herein refers to a straight-chain or branched alkyl group having 1 to 8 carbon (s). For example, methyl, ethyl, n-propyl, isopropyl, n-butyl, isobutyl, sec-butyl, tert-butyl, n-pentyl, isopentyl, neopentyl, tert-pentyl, n-hexyl, isohexyl and the like can be exemplified. A straight-chain or branched alkyl group having 1 to 4 carbon(s) such as methyl, ethyl, n-propyl, isopropyl, n-butyl, isobutyl, sec-butyl, tert-butyl or the like is preferred.
The term "aralkyl" used herein refers to the afore-defined alkyl substituted with phenyl or naphthyl, for example, benzyl, phenethyl, naphthylmethyl and the like.
The term "aryl" used herein refers to an aromatic carbocyclic group of monocycle or condensed ring having 6 to 14 carbon(s). For example, phenyl, naphthyl, phenanthryl and the like are recited. Phenyl is particularly preferred.
The term "heteroaryl" used herein refers to 5- to 8-membered aromatic heterocyclic group having one to four oxygen atom, sulfur atom and/or nitrogen atom in a ring, or an aromatic heterocyclic group in which 5- to 8-membered aromatic heterocycle is condensed with one to four 5- to 8-membered aromatic carbocycle or other 5-to 8-membered aromatic heterocycle, and may have a bonding hand at any position which allows substitution. The bonding hand may be located at either carbon atom or nitrogen atom in a ring. For example, thienyl, furyl, pyrrolyl, imidazolyl, pyrazolyl, triazolyl, tetrazolyl, oxazolyl, isoxazolyl, thiazolyl, isothiazolyl, pyridyl, pyridazinyl, pyrimidinyl, furazanyl, pyrazinyl, thiadiazolyl, oxadiazolyl, benzofuryl, benzothienyl, benzimidazolyl, benzothiazolyl, indolyl, dibenzofuryl, quinolyl, isoquinolyl, cinnolyl, quinazolyl, quinoxalyl, phthaladinyl, puryl, puteridinyl, carbazolyl, phenanthridinyl, acrydinyl, phanazinyl, 1,10-phenanthronitrile, isoindolyl, 1H-indazolyl, indolidinyl and the like can be recited.
Alkyl moiety in alkyloxy is as same as the alkyl as described above.
Halogen means fluorine, chlorine, bromine or iodine.
Examples of substituent in optionally substituted aralkyl, optionally substituted aryl, and optionally substituted heteroaryl include the afore-defined alkyl, afore-defined alkyloxy, halogen and the like.

In the following, explanation will be made on a method of producing a compound shown by formula: 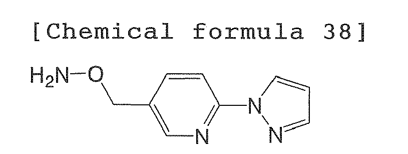 using a compound of the present inveniton.

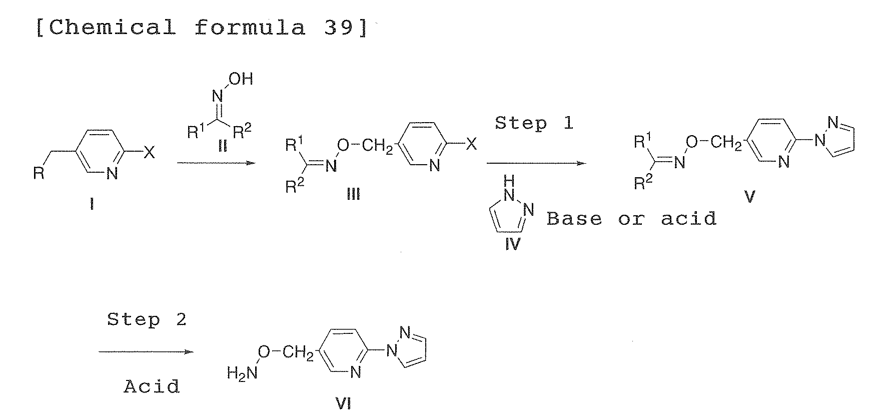

### Step 1

This is a step of reacting a compound shown by formula III (R¹ and R² each independently represent hydrogen, alkyl, optionally substituted aralkyl, optionally substituted aryl or optionally substituted heteroaryl, X is halogen) with a compound shown by formula IV in the presence of base or acid, to obtain a compound shown by formula V.
As the base, sodium hydride, lithium hydride, sodium hydroxide, potassium hydroxide, triethylamine, DBU(1,8-diazabicyclo[5.4.0]undec-7-ene), sodium methoxide, potassium tert-butoxide and the like may be used. An amount of the base may be, for example, 1.05 to 1.5 equivalents, relative to the compound shown by formula III.
As the acid, p-toluenesulfonic acid, benzenesulfonic acid, sulfuric acid, acetic acid, hydrochloric acid and the like may be used. An amount of the acid may be for example, 1.05 to 1.5 equivalents, relative to the compound shown by formula III. As the solvent, dimethylsulfoxide, dimethylformamide, dimethylacetamide, tetrahydrofuran, N-methylpyrrolidinone, N,N-dimethylimidazolydinone, diglyme, triglyme and the like may be used. Reaction may be carried out at about 0 to 150°C, for example, at 120°C. The reaction may be carried out in the presence of a catalytic amount (e.g., 0.1 to 0.2 equivalents) of sodium para-toluenesulfonate, sodium iodide, potassium iodide, or sodium paratoluenesulinate.
The compound shown by formula III may be obtained by reacting a compound shown by formula I (for example, R is halogen, alkylsulfonyloxy or arylsulfonyloxy, X is halogen) with a compound shown by formula II (R¹ and R² each independently represent hydrogen, alkyl, optionally substituted aralkyl, optionally substituted aryl or optionally substituted heteroaryl) in the presence of base in a solvent such as dimethylformamide, dimethylsulfoxide, N-methylpyrrolidinone or the like. As the base, for example, sodium hydroxide, potassium hydroxide, sodium carbonate, potassium carbonate or the like may be used. The solvent may contain water. As the compound shown by formula III, compounds in which R¹ and R² each independently represent hydrogen, alkyl, optionally substituted aralkyl, optionally substituted aryl or optionally substituted heteroaryl, and X is halogen can be recited. For example, compounds in which R¹ and R² are methyl, compounds in which R¹ is ethyl and R² is methyl, compounds in which R¹ is phenyl and R² is methyl and the like may be used.

### Step 2

This is a step of hydrolyzing a compound shown by formula V (R¹ and R² each independently represent hydrogen, alkyl, optionally substituted aralkyl, optionally substituted aryl or optionally substituted heteroaryl) in the presence of acid, to obtain a compound shown by formula VI.
As the acid, hydrochloric acid (concentrated HCl diluted HCl), sulfuric acid, phosphoric acid or nitric acid may be used. As the solvent, alcohol, acetic acid, ethyl acetate, acetonitrile, dimethylformamide or the like may be used. Particularly preferred is to carry out hydrolysis using diluted sulfuric acid in water solvent. Reaction may be carried out at -20 to 150°C, for example, at room temperature.

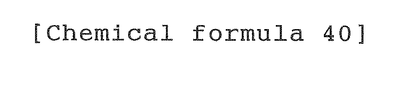

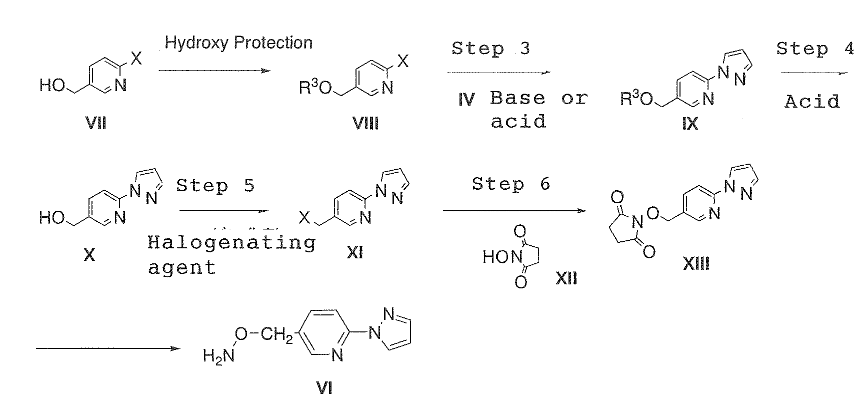

### Step 3

This is a step of reacting a compound shown by formula VIII (R³ is protective group of hydroxy, and X is halogen) with a compound shown by formula IV in the presence of base, to obtain a compound shown by formula IX.
As the base, sodium hydride, lithium hydride, sodium hydroxide, potassium hydroxide, potassium tert-butoxide, sodium methoxide or the like may be used. The base may be used, for example, in an amount of 1.05 to 1.5 equivalents, relative to the compound shown by formula VIII.
As the acid, p-toluenesulfonic acid, benzenesulfonic acid, sulfuric acid, acetic acid, hydrochloric acid or the like may be used.
As the solvent, dimethylsulfoxide, dimethylformamide, dimethylacetamide, tetrahydrofuran, N-methylpyrrolidinone, N,N-dimethylimidazolydinone, diglyme, triglyme or the like may be used.
The reaction may be carried out at 0 to 150°C, for example, at 120°C. A catalytic amount (for example, 0.1 to 0.2 equivalents) or sodium paratoluenesulinate may be used.
As the protective group of hydroxy, tetrahydropyranyl, methoxymethyl, 1-methoxy-1-methylethyl, 1-othoxyethyl, methyl, benzyl, substituted benzyl and the like are recited. Particularly preferred is tetrahydropyranyl.
As the halogen, fluorine, chlorine, bromine, iodine and the like can be recited. Particularly preferred is chlorine.

### Step 4

This is a step of hydrolyzing a compound shown by formula IX (R³ represents a protective group of hydroxy) in the presence of acid or hydrogenolyzing in hydrogen atmosphere, to obtain a compound shown by formula X.
As the acid, hydrochloric acid (concentrated HCl, diluted HC1), sulfuric acid, nitric acid, acetic acid, acidic ion exchange resin, solid acid or the like may be used. Particularly preferred is concentrated HCl.
As the solvent, alcohol may be used. For example, methyl alcohol, ethyl alcohol, or isopropyl alcohol may be used.
Particularly preferred is isopropyl alcohol. The reaction may be carried out at 0 to 50°C, for example, at room temperature.

### Step 5

This is a step of reacting a compound shown by formula X with a halogenating agent, to obtain a compound shown by formula XI.
As the halogenating agent, thionyl chloride, thionyl bromide, phosphorus tribromide, oxalyl chloride or the like may be used. Particularly preferred is thionyl chloride. As the solvent, benzene, toluene, dimethylformamide, ethyl acetate, acetonitrile or the like may be used. These solvents may be used in mixture. For example, a mixed solvent of toluene and dimethylformamide may be used. The reaction may be carried out at 0 to 50°C for example, at room temperature.

### Step 6

This is a step of reacting a compound shown by formula XI (X is halogen) with a compound shown by formula XII , to obtain a compound shown by formula XIII.
As the solvent, dimethylsulfoxide, dimethylformamide, dimethylacetamide, tetrahydrofuran, N-methylpyrrolidinone, N,N-dimethylimidazolydinone, diglyme, triglyme or the like may be used. The reaction is preferably carried out in the presence of base. As the base, DBU(1,8-diazabicyclo[5.4.0]undec-7-ene), triethylamine, sodium hydroxide aqueous solution, lithium hydroxide aqueous solution or the like may be used. The reaction may be carried out at 0 to 50°C, for example, at room temperature.

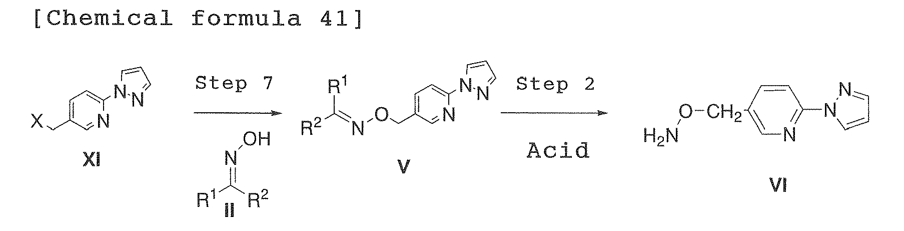

### Step 7

This is a step of reacting a compound shown by formula XI (X is halogen or the like) with a compound shown by formula II in the presence of base, to obtain a compound shown by formula V.
As the base, sodium hydride, lithium hydride, sodium hydroxide, potassium hydroxide, triethylamine, DBU(1,8-diazabicyclo[5.4.0]undec-7-ene), sodium methoxide, potassium tert-butoxide or the like may be used. The base may be used, for example, in an mount of 1.05 to 1.5 equivalents, relative to the compound shown by formula XI. As the solvent, dimethylsulfoxide, dimethylformamide, dimethylacetamide, tetrahydrofuran, N-methylpyrrolidinone, N.N-dimethylimidazolydinone, diglyme, triglyme or the like may be used. The reaction may be carried out at 0 to 50°C, for example, at room temperature.

A compound shown by formula: 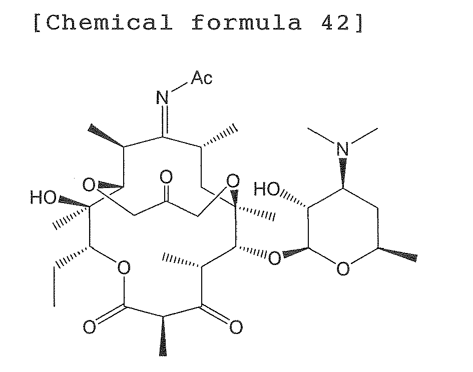 may be produced in accordance with a method described in Patent document 1.
A compound shown by formula: 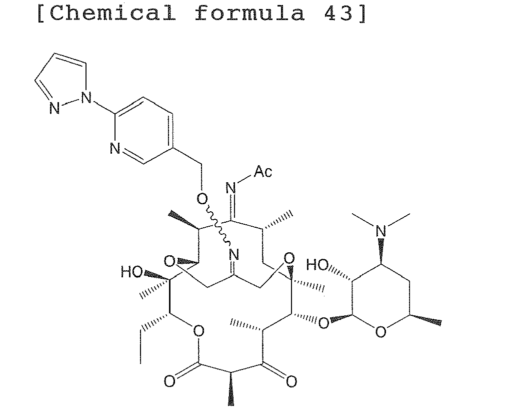 may be produced by reacting a compound shown by formula VI with a compound shown by formula: 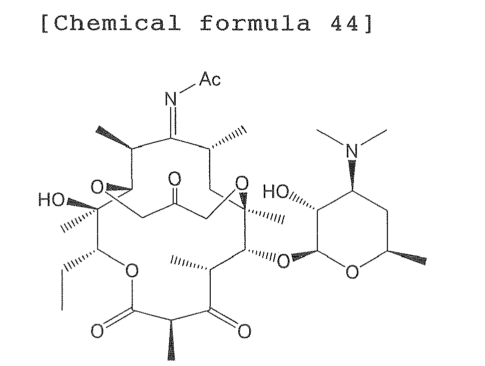 in the presence of acid or base. The conditions and the like may follow Patent document 1.

The present invention embraces salts of compounds shown by formula: 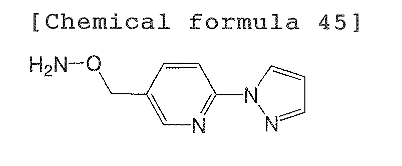 As the salt, hydrochloric acid salt, sulfuric acid salt, trichloroacetic acid salt, trifluoroacetic acid salt, methanesulfonic acid salt, p-toluenesulfonic acid, benzenesulfonic acid, perchloric acid, hydrobromic acid, benzoic acids, nitric acid, acetic acid, carbonic acid, oxalic acid, phosphoric acid and the like are recited, and hydrochloric acid salt, sulfuric acid salt, trichloroacetic acid salt or trifluoroacetic acid is particularly preferred. Hydroxylamine having a melting point of 42.5-42.6°C, by itself is low in melting point, poor in stability and poor in workability in production. For addressing this, as is the salt of the present invention, a salt having high melting point (for example, salt having a melting point of 100°C or higher, for example, sulfuric acid salt: m.p.= 197.8-197.9°C, hydrochloric acid salt m.p.= 207.3-207.4°C, trichloroacetic acid salt: m.p.=127.2-127.3°C, trifluoroacetic acid salt m.p.= 117.7°C) is used to achieve improvement in productivity in production of antimicrobial agent.
Further, the present invention also embraces the following compounds. The following compound has excellent characteristics as crystals and is easy to be crystallized from water-containing solvent in spite of its low melting point (when X is chloro, m.p.= 46-47.5°C). Therefore, the process using this compound is particularly suited for industrial production. X is halogen, and particularly preferably chloro. Compound shown by: 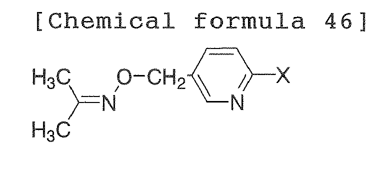 (wherein X is halogen)

In the following, Examples will be described, however, it is to be noted that the present invention will not be limited to these Examples.

### Examples

### [Reference example 1]

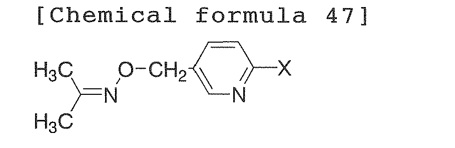

To a solution of 500 mg (5.74 mmol) of 2-butanone oxime and 930 mg (5.74 mmol) 2-chloro-5-chloromethylpyridine in 1.3 mL DMF, 610 mg (7.32 mmol) of 48% sodium hydroxide aqueous solution was added under cooling on ice, and stirred for 3 hours at room temperature. The reaction mixture was added with iced water, and extracted with toluene. The obtained toluene layer was washed with saturated saline, dried over anhydrous magnesium sulfate, and toluene was distilled off under reduced pressure, to give 970 mg of residue (yield 80%).
E isomer: ¹H NMR (CDCl₃, 300 MHz) d1.07 (t, 3H, J= 7.5 Hz), 1.85 (s, 3H), 2.18 (q, 2H, J= 7.5 Hz), 5.04 (s, 2H), 7.31 (d, 1H, J= 7.8 Hz), 7.63-7.68 (m, 1H), 8.36-8.39 (m, 1H); TLC (silicagel, toluene/AcOEt= 10/1) Rf= 0.45.
Z isomer: ¹H NMR (CDCl₃, 300 MHz) d1.04 (t, 3H, J= 7.5 Hz), 1.84 (s, 3H), 2.34 (q, 2H, J= 7.5 Hz) 5.03 (s, 2H), 7.31 (d, 1H, J= 8.7 Hz), 7.62-7.67 (m, 1H), 8.36-8.38 (m, 1H) ; TLC (silicagel, toluene/AcOEt= 10/1) Rf= 0.40

### [Reference example 2]

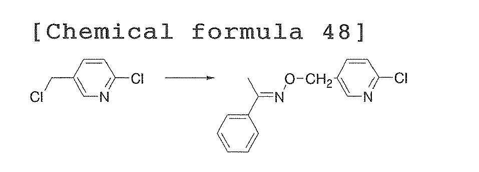

To a solution of 680 mg (5.00 mmol) of acetophenone oxime and 930 mg (5.00 mmol) of 2-chloro-5-chloromethylpyridine in 1.1 mL DMF, 540 mg (6.48 mmol) of 48% sodium hydroxide aqueous solution was added under cooling on ice, and stirred for 4 hours at room temperature. The reaction mixture was added with iced water, and extracted with toluene. The obtained toluene layer was washed with saturated saline, dried over anhydrous magnesium sulfate, and toluene was distilled off under reduced pressure, to give 1.25 g of residue. The obtained residue was subjected to silica-gel chromatography, and eluted with 4%ethyl acetate-toluene solvent, to give 1.07 g of an objective product (yield 82%)
¹H NMR (CDCl₃, 300 MHz) d2.25 (s, 3H), 5.21 (s, 2H), 7.31-7.39 (m, 4H), 7.57-7.65 (m, 2H), 7.71 (dd, 1H, J= 2.4, 8.1 Hz), 8.43-8.47 (m, 1H)
TLC (silicagel, toluene/AcOEt= 10/1) Rf= 0.5.

### [Reference example 3]

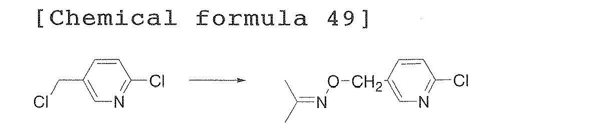

Under cooling on ice, 48% sodium hydroxide aqueous solution 3.34 g (40.1 mmol) was added with 7.5 mL of DMF and 2.48 g (33.9 mmol) of acetone oxime, and stirred at room temperature. The reaction mixture was added with 5.00 g (30.9 mmol) of 2-chloro-5-chloromethylpyridine, and stirred for another one hour at room temperature. The resultant reaction mixture was added with 50 g of iced water and 4.5 g NaCl (table salt), and precipitated crystals were collected by filtering, to give 4.80 g of an objective product (yield 78%).
¹H NMR(CDCl₃, 300 MHz) d 1.86 (s, 3H), 1.87 (s, 3H), 5.04 (s, 2H), 7.31 (d, 1H, J= 8.3 Hz), 7.65 (dd, 1H, J= 2.3, 8.3 Hz), 8.37 (d, 1H, J= 2.3 Hz)
TLC (silicagel, toluene/AcOEt= 10/1) Rf= 0.35
m.p.= 46 - 47.5 °C.

### [Example 1]

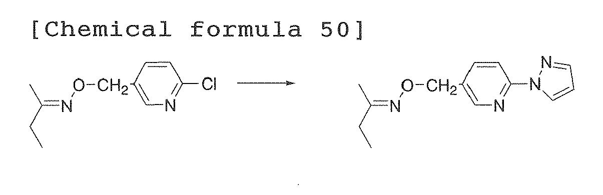

Under cooling on ice, a solution of 1.97 g(45.1mmol) of sodium hydride (when content is 60%) in 8 mL DMF was added dropwise with a solution of 3.33 g (48.9 mmol) of pyrazole in 8 mL DMF, and a solution of 8.00 g (37.6 mmol) of 0-(2-chloropyridine-5-yl)methyl 2-butanone oxime in 8 mL DMF in a sequential manner. Then the mixture was stirred under heating at 120°C for 3.5 hours. Then the reaction mixture was added with water, and extracted with ethyl acetate. The obtained ethyl acetate layer was washed with saturated saline, dried over anhydrous magnesium sulfate, and ethyl acetate was distilled off under reduced pressure. The obtained residue was subjected to silica-gel chromatography, and eluted with 10% ethyl acetate-hexane solution, to give 7.18 g of objective product (yield 78%).
E isomer: ¹H NMR (CDCl₃, 300 MHz) d 1.07 (t, 3H, J= 7.8 Hz), 1.86 (s, 3H), 2.18 (q, 2H, J= 7.8 Hz), 5.09 (s, 2H), 6.46 (dd, 1H, J= 1.5, 2.1 Hz), 7.74 (d, 1H, J= 1.5 Hz), 7.81-7.86 (m, 1H, J= 2.4, 8.7 Hz), 7.97 (d, 1H, J= 8.7 Hz), 8.41 (d, 1H), 8.61 (d, 1H, J= 2.7 Hz);
TLC (silicagel, toluene/AcOEt= 5/1) Rf= 0.50.
Z isomer: ¹H NMR (CDCl₃, 300 MHz) d1.07 (t, 3H, J= 7.8 Hz), 1.84 (s, 3H), 2.35 (q, 2H, J= 7.8 Hz), 5.07 (s, 2H), 6.46 (dd, 1H, J= 1.5, 2.1 Hz), 7.74 (d, 1H, J= 1.5 Hz), 7.81-7.86 (m, 1H, J= 2.4, 8.7 Hz), 7.97 (d, 1H, J= 8.7 Hz), 8.41 (d, 1H), 8.61 (d, 1H, J= 2.7 Hz);
TLC (silicagel, toluene/AcOEt= 5/1) Rf= 0.45.

### [Example 2]

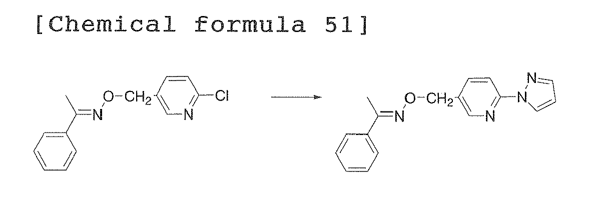

After adding 152 mg (3.48 mmol) of 55% sodium hydride to a solution of 274 mg (4.02 mmol) of pyrazole in 3.5 mL DMF at room temperature, the mixture was stirred for 5 minutes at room temperature. After adding 48 mg (0.27 mmol) of sodium p-toluene sulfinate, and 704 mg (2.70 mmol) of O-(2-chloropyridine-5-yl)methyl acetophenone oxime, the resultant mixture was stirred under heating at 120°C for 5.5 hours. The reaction mixture was added with iced water, and extracted with ethyl acetate. The obtained toluene layer was washed with saturated saline, dried over anhydrous magnesium sulfate, and toluene was distilled off under reduced pressure, to give 839 mg of residue. The obtained residue was subjected to silica-gel chromatography, and eluted with 3% ethyl acetate-toluene solvent, to give 461 mg of an objective product (yield 53%).
¹H NMR (CDCl₃, 300 MHz) d2.26 (s, 3H), 5.25 (s, 2H), 6.46 (dd, 1H, J= 1.7, 2.6 Hz), 7.33-7.39 (m, 4H), 7.59-7.65 (m, 2H), 7.74 (dd, 1H, J= 0.8, 1.7 Hz), 7.88 (dd, 1H, J= 2.1, 8.4 Hz), 7.99 (dd, 1H, J= 2.4, 8.4 Hz), 8.46-8.48 (m, 1H), 8.57 (dd, 1H, J= 0.8, 2.6 Hz);
TLC (silicagel, toluene/AcOEt= 10/1) Rf= 0.6.

### [Example 3]

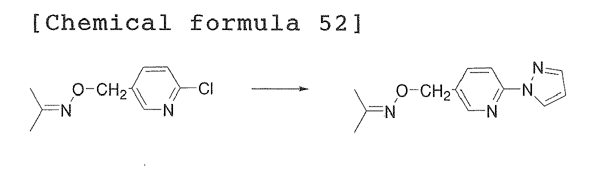

To a solution of 514 mg (7.55 mmol) of pyrazole in 8 mL diglyme, 1.26 g (6.53 mmol) of 28% sodium methoxide was added at room temperature, and then methanol and diglyme (total 4 mL) were distilled off under reduced pressure. The residue of distillation was added with 1.00 g (5.03 mmol) of O-(2-chloropyridine-5-yl)methyl acetone oxime at room temperature, and stirred for 6 hours and 20 minutes under heating at 120°C. The obtainable reaction mixture was added with 15 mL of iced water, and the precipitated crystals were collected by filtering, to give 860 mg of an objective product (yield 74%).
To a solution of 1.99 g (10.0 mmol) of O-(2-chloropyridine-5-yl)methyl acetone oxime and 681 mg (10.0 mmol) of pyrazole in 6 mL diglyme, 1.90 g (10.0 mmol) of p-toluenesulfonic acid monohydrate was added at room temperature, and stirred for 10 hours under heating at 100°C. After reaction, the mixture was added with iced water, and extracted with ethyl acetate. The obtained organic layer was washed with saturated saline, and ethyl acetate solvent was distilled off under reduced pressure. The obtained residue was subjected to silica-gel chromatography, and eluted with ethyl acetate/hexane(1/20), to give 1.22 g of an objective product (yield 53%).
¹H NMR (CDCl₃, 300 MHz) d1.87 (s, 3H), 1.88 (s, 3H), 5.08 (s, 2H), 6.46 (dd, 1H, J= 1.5, 2.4 Hz), 7.73 (d, 1H, J= 1.5 Hz), 7.81 (dd, 1H, J= 2.4, 8.7 Hz), 7.96 (dd, 1H, J= 0.6, 8.7 Hz), 8.39 (d, 1H, J= 2.4 Hz), 8.56 (dd, 1H, J= 0.6, 2.4 Hz); TLC (silicagel, toluene/AcOEt= 4/1) Rf= 0.5;
m.p.= 57-59°C.

### [Example 4]

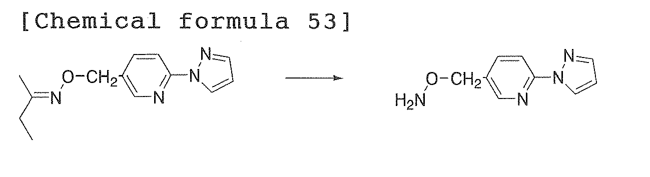

To a solution of 180.7 mg (1.84 mmol) of 98% sulfuric acid in 900 µL water, 50.0 mg (0.21 mmol) of O-[2-(pyrazole-1-yl)pyridine-5-yl]methyl 2-butanone oxime was added and stirred for 2 hours under reduced pressure of 150 Torr at 60°C. The reaction solution was washed with toluene, and added with 1 M sodium hydroxide aqueous solution. This was then extracted with toluene. The obtained toluene layer was washed with saturated saline, dried over anhydrous magnesium sulfate, and toluene was distilled off under reduced pressure, to give 25.7 mg of residue (yield 66%).
¹H NMR (CDCl₃, 300 MHz) d 4.71 (s, 2H), 5.46 (brs, 2H), 6.47 (dd, 1H, J= 1.7, 2.4 Hz), 7.74 (dd, 1H, J= 0.8, 1.7 Hz), 7.83 (dd, 1H, J= 2.4, 8.4 Hz), 7.98 (dd, 1H, J= 0.9, 8.4 Hz), 8.40 (dd, 1H, J= 0.8, 2.4 Hz), 8.57 (dd, 1H, J= 0.9, 2.4 Hz); m.p.= 42.5-42.6°C;
TLC (silicagel, CHCl₃/MeOH= 100/3) Rf= 0.4.

### [Example 5]

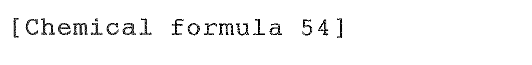

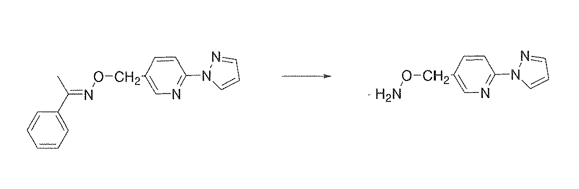

To 500 µL of mixture solution of 18% hydrochloric acid: acetic acid (5:1), 25.0 mg (0.09 mmol) of O-[2-(pyrazole-1-yl)pyridine-5-yl]methyl acetophenone oxime was added and stirred for one hour at 80°C. The reaction mixture was added with sodium hydroxide aqueous solution, and extracted with ethyl acetate. The obtained ethyl acetate layer was washed with saturated saline, dried over anhydrous magnesium sulfate, and ethyl acetate was distilled off under reduced pressure. The obtained residue was subjected to silica-gel chromatography, and developed with 3% methanol-chloroform solvent, to give 12.1 mg of an objective product(yield 74%).
¹H NMR (CDCl₃, 300 MHz) d4.71 (s, 2H), 5.46 (brs, 2H), 6.47 (dd, 1H, J= 1.7, 2.4 Hz), 7.74 (dd, 1H, J= 0.8, 1.7 Hz), 7.83 (dd, 1H, J= 2.4, 8.4 Hz), 7.98 (dd, 1H, J= 0.9, 8.4 Hz), 8.40 (dd, 1H, J= 0.8, 2.4 Hz), 8.57 (dd, 1H, J= 0.9, 2.4 Hz); m.p.= 42.5-42.6 °C;
TLC (silicagel, CHCl_{3/}MeOH= 100/3) Rf= 0.4.

### [Example 6]

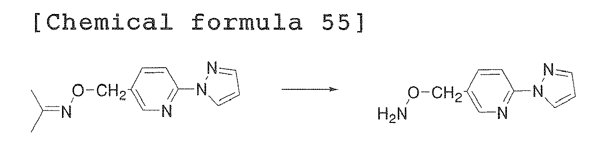

To a solution of 180.7 mg (1.84 mmol) of 98% sulfuric acid in 900 µL water, 50.0 mg (0.22 mmol) of O-[2-(pyrazole-1-yl)pyridine-5-yl]methyl acetone oxime was added and stirred for 2 hours under reduced pressure of 400 Torr at 90°C. The reaction solution was washed with toluene, and added with 1 M sodium hydroxide aqueous solution. This was then extracted with toluene. The obtained toluene layer was washed with saturated saline, dried over anhydrous magnesium sulfate, and toluene was distilled off under reduced pressure, to give 37.0 mg of residue (yield 90%).
¹H NMR (CDCl₃, 300 MHz) d4.71 (s, 2H), 5.46 (brs, 2H), 6.47 (dd, 1H, J= 1.7, 2.4 Hz), 7.74 (dd, 1H, J= 0.8, 1.7 Hz), 7.83 (dd, 1H, J= 2.4, 8.4 Hz), 7.98 (dd, 1H, J= 0.9, 8.4 Hz), 8.40 (dd, 1H, J= 0.8, 2.4 Hz), 8.57 (dd, 1H, J= 0.9, 2.4 Hz); m.p.= 42.5-42.6°C;
TLC (silicagel, CHCl₃//MeOH= 100/3) Rf= 0.4.

### [Reference example 4]

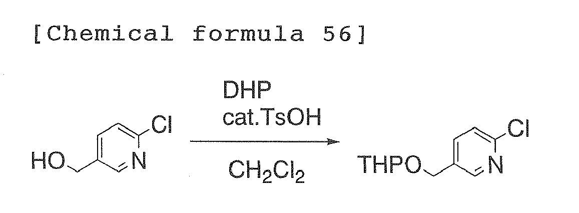

To a solution of hydroxymethyl form product (10.0 g, 69.65 mmol) in toluene (10 mL), 3,4-dihydro-2H-pyran (7.03 g, 83. 58 mmol) and p-toluenesulfonic monohydrate (133 mg, 0.70 mmol) were added sequentially at room temperature. At the same temperature, the mixture was stirred for one hour, and added with 48% sodium hydroxide aqueous solution (116 mg, 1.40 mmol). Water (20 mL) was added, and the resultant mixture was extracted with ethyl acetate (30 mL x 2). The organic layers were combined, washed with water (20 mL), and then concentrated under reduced pressure, to give a crude THP form product.
¹H NMR (CDCl₃, 300 MHz) d8.37 (d, J= 2.7 Hz, 1H), 7.83 (dd, J= 2.4, 8.1 Hz, 1H), 7. 17 (d, J= 7.2 Hz, 1H), 4.77 (d, J= 12.3 Hz, 1H), 4.74 (dd, J= 3.0, 3.3 Hz, 1H), 4.54 (d, J= 12.3 Hz, 1H), 3.91 (m, 1H), 3.56 (m, 1H), 1.48-1.92 (m, 6H);

### [Example 7]

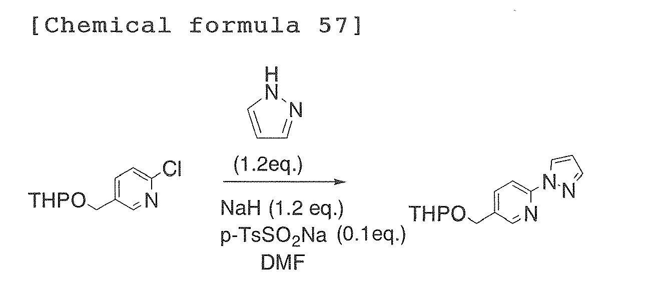

After adding the crude THP form product (13.70g, 60.17 mmol) obtained in Reference example 4 to a solution of pyrazole sodium salt prepared in advance from sodium hydride (1.733 g, 72.20 mmol) and pyrazole (6.58 g, 78.22 mmol) in DMF (69 mL), the mixture was heated to 120 degrees. After stirring for 6 hours at 120 degrees, water (68.5 mL) was added at room temperature, and the generated mixture was extracted with ethyl acetate (99 mL x 2). The organic layers were combined, washed with water (137 mL x 2), and concentrated under reduced pressure, to give a crude pyrazole form product.
¹H NMR (CDCl₃, 300 MHz) d8.56 (dd, J= 0.7, 2.6 Hz, 1H), 8.40 (dd, J= 0.7, 2.3 Hz, 1H), 7.97 (dd, J= 0.7, 8.6 Hz, 1H), 7.83 (dd, J= 2.0, 8.6 Hz, 1H), 7.74 (dd, J= 0.7, 1.0 Hz, 1H), 6.47 (dd, J= 1.6, 2.6 Hz, 1H), 4.82 (d, J= 12.2 Hz, 1H), 4.74 (dd, J= 3.3, 3.6 Hz, 1H), 4.49 (d, J= 12.2 Hz, 1H), 3.87 (ddd, J= 3.3, 8.1, 10.8 Hz, 1H), 3. 55 (m, 1H), 1.48-1.92 (m, 6H) ; ¹³C NMR (CDCl₃, 75MHz) d147.4, 142.0, 131.5, 127.0, 119.1, 112.0, 107.7, 97.9, 65.9, 62.2, 30.4, 25.3, 19.3.

### [Example 8]

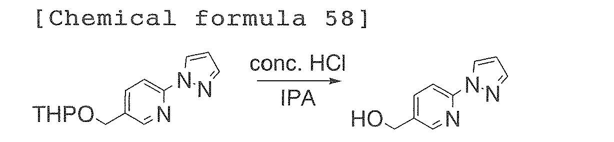

To a solution of the obtained crude pyrazole form product in IPA (69 mL), 12 N HCl (10 mL, 120.34 mmol) was added at 30 degrees. After stirring for 5 hours at this temperature, solvent was concentrated by about 50 mL under reduced pressure, and IPA (14 mL) was added. This was then stirred at 0 degree, and added dropwise with toluene (14 mL). The obtained crystals were collected by filtering, to give Pyr-Py-OH form hydrochloric acid salt (5.28 g, 42% for 3 steps).
¹H NMR (CD₃OD, 300 MHz) d8.68 (dd, J= 0.7, 3.0 Hz, 1H), 8.48 (dd, *J=* 0.7, 2.0 Hz, 1H), 8.29 (dd, *J*= 2.0, 8.6 Hz, 1H), 8.16 (dd, *J=* 0.7, 8.6 Hz, 1H), 7.93 (d, *J=* 1.3 Hz, 1H), 6.69 (dd, *J=* 2.0, 3.0 Hz, 1H), 4.75 (s, 2H).

### [Example 9]

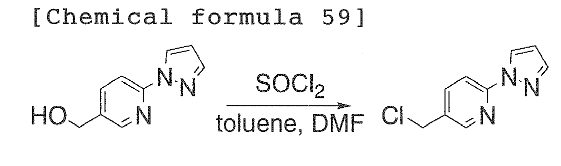

To a solution of Pyr-Py-OH form hydrochloric acid salt (0.50 g, 2.36 mmol) in ethyl acetate (2.5 mL) and DMF (2.5 mL), thionyl chloride (0.51 g, 4.25 mmol) was added at room temperature. After stirring for 30 minutes at this temperature, about 3 mL of solvent was distilled off under reduced pressure. Water (5 mL) was added and the generated mixture was extracted with ethyl acetate (5 mL x2). The organic layers were combined, washed with water (5 mL), and concentrated under reduced pressure. The residue was added with toluene (14 mL), and concentrated again under reduced pressure, to give a chloro form product (453 mg, 99%).
¹H NMR (CDCl₃, 300 MHz) d8.60 (dd, J= 0.7, 2.6 Hz, 1H), 8.47 (d, J= 2.0 Hz, 1H), 8.00 (dd, J= 2.3, 8.6 Hz, 1H), 7.94 (dd, J= 0.7, 8.2 Hz, 1H), 7.77 (d, J= 1.0 Hz, 1H), 6.54 (dd, J= 1.7, 2.6 Hz, 1H), 4.63 (s, 2H).

### [Example 10]

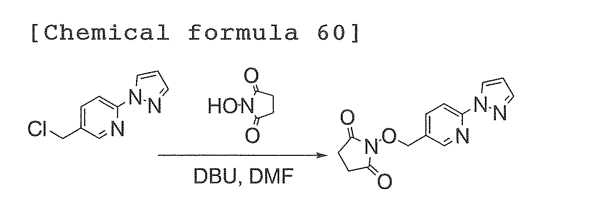

To a solution of hydroxysuccinimide (13.37 g, 116.20 mmol) in DMF (75 mL), DBU (17.69 g, 116.20 mmol), and a solution of chloro form product (15.0 g, 77.47 mmol) in DMF (75 mL) were sequentially added at room temperature. After stirring for one hour at this temperature, water (100 mL) was added, and the generated mixture was extracted with ethyl acetate (500 mL) . The organic layers were combined, washed with water (100 mL x 2), again concentrated under reduced pressure. The residue was added with toluene (100 mL), and concentrated again under reduced pressure, and the resultant residue was added with hexane (100 mL) at 0 degree. The obtained crystals were collected by filtering to give a succinimide form product (13.92 g, 66%).
¹H NMR (CDCl₃, 300 MHz) d8.57 (dd, J= 0.7, 2.6 Hz, 1H), 8.46 (m, 1H), 8.03 (s, 1H), 8.02 (s, 1H), 7.75 (m, 1H), 6.48 (dd, J= 1.7, 2.6 Hz, 1H), 5.15 (s, 2H), 2.70 (s, 4H); ¹³C NMR (CDCl₃, 75 MHz) d171.0, 152.2, 149.1, 142.5, 140.5, 127.3, 126.9, 112.2, 108.1, 75.4, 25.4, 20.0.

### [Example 11]

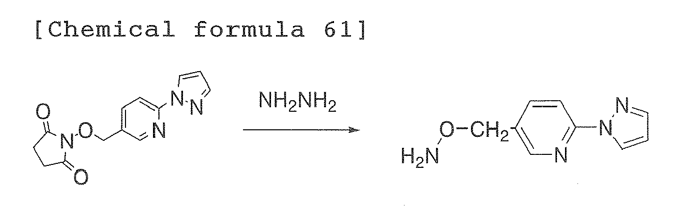

To a solution of succinimide form product (15.80 g, 58.03 mmol) in methanol (237 mL), hydrazine monohydrate (5.7 mL) was added at room temperature, and stirred for 3 hours at 45 degrees. After filtering precipitated crystals, the filtrate was concentrated by about 190 mL. The resultant concentrated solution was added with ethyl acetate, washed with aqueous saturated sodium carbonate (50 mL), and saturated saline (50 mL), and then immediately concentrated under reduced pressure. The residue was added with toluene (51 mL), concentrated again under reduced pressure, and the obtained residue was added with hexane (51 mL) at 0 degree. The obtained crystals were collected by filtering to give a hydroxyamino form product (8.56 g, 78%).
m.p. 43.0-43.5°C, ¹H NMR (CD₃OD, 300 MHz) d 8.59 (dd, *J=* 0.7, 2.6 Hz, 1H), 8.41 (dd, *J=* 0.7, 2.3 Hz, 1H), 7.932 (d, *J=* 0.7 Hz, 1H), 7.926 (s, 1H), 7.75 (dd, *J=* 0.7, 1.6 Hz, 1H), 6.53 (dd, *J=* 2.0, 2.6 Hz, 1H), 4.71 (s, 2H).

### [Example 12]

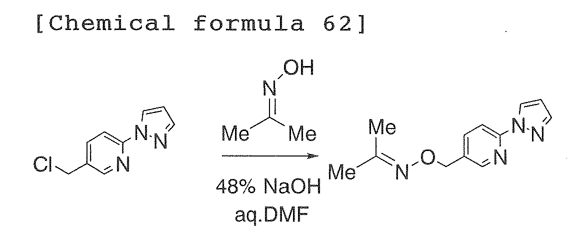

Under cooling on ice, 16.1 g of DMF and 2.01 g (23.1 mmol) of acetone oxime were added to 1.92 g (23.1 mmol) of 48% sodium hydroxide aqueous solution, and stirred at room temperature. This reaction mixture was added with 3.72 g (19.2 mmol) of 3-chloromethyl-6-(pyrazole-1-yl)pyridine, and stirred for another one hour at room temperature. The obtained reaction mixture was added with 11.2 g of iced water, and extracted with ethyl acetate. After washing the ethyl acetate layer with water, ethyl acetate was distilled off under reduced pressure, to give 4.69 g of an objective product (yield 79%).
¹H NMR (CDCl₃, 300 MHz) d1.86 (s, 3H), 1.87 (s, 3H), 5.04 (s, 2H), 7.31 (d, 1H, J= 8.3 Hz), 7.65 (dd, 1H, J= 2.3, 8.3 Hz), 8.37 (d, 1H, J= 2.3 Hz);
TLC (silicagel, toluene/AcOEt= 10/1) Rf= 0.35;
m.p.= 46-47.5°C

### [Example 13]

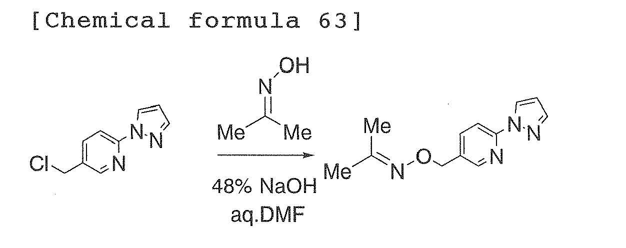

To a solution of 500 mg (2.63mmol) of O-[2-(pyrazole-1-yl)pyridine-5-yl]methylhydroxylamine in 2 mL ethanol, 143 µL (2.63 mmol) of sulfuric acid was added dropwise. After sequentially adding 4 mL of water and 4 mL of ethanol, precipitated crystals were collected by filtering, to give 592.2 mg of an objective product (yield 82.1%).
¹H NMR (DMSO 300 MHz) d4.86 (s, 2H), 6.60 (dd, 1H, J= 1.8,2.7 Hz), 7.84 (dd, 1H, J= 1.2 Hz), 7.93 (m, 2H), 8.47 (dd, 1H, J= 1.5 Hz), 8.64 (dd, 1H, J= 2.7 Hz)
m.p.= 197.8-197.9°C

| Element analysis | | | | | |
|---|---|---|---|---|---|
| C₁₈H₂₂N₈O₆S | Calc.: | H(%) 4.63, | C(%) 45.18, | N(%) 23.42, | S(%) 6.70 |
| | Found: | H(%) 4.31, | C(%)45.24, | N(%) 23.39, | S(%) 6.56 |

### [Example 14]

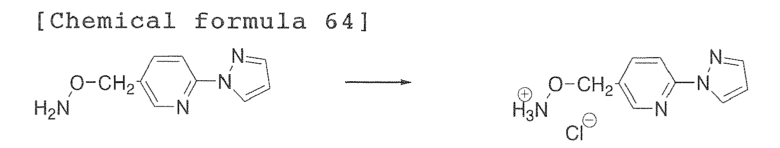

To a solution of 500 mg (2.63 mmol) of O-[2-(pyrazole-1-yl)pyridine-5-yl]methylhydroxylamine in 2 mL ethanol, 219 µL (2.63 mmol) of 12N hydrochloric acid was added dropwise. Precipitating white solids were dissolved by adding 2 mL of water. Then 6 mL of ethanol was added dropwise, and crystals that are precipitated as a result of ice cooling were collected by filtering, to give 205.1 mg of an objective product (yield 36.7%).
¹H NMR (DMSO 300 MHz) d5.14 (brs, 2H) 6.60 (dd, 1H, J= 1.2 Hz), 7.86 (dd, 1H), 8.00 (m, 3H), 8.53 (dd, 1H, J= 1.5 Hz), 8.65 (dd, 1H, J= 2.4 Hz)
m.p.= 207.3-207.4°C

| Element analysis | | | | | |
|---|---|---|---|---|---|
| C₈H₉ClN₄O | Calc.: | H(%) 4.27, | C(%) 45.19, | N(%) 26.35, | Cl(%) 16.67 |
| | Found: | H(%) 4.67, | C(%) 47.46, | N(%) 24.63, | Cl(%) 15.20 |

### [Example 15]

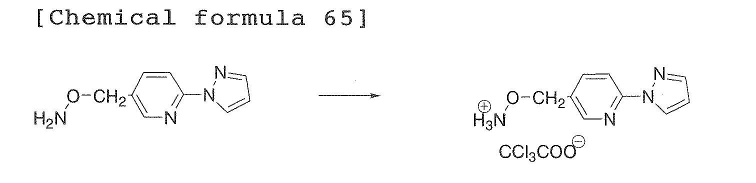

To a solution of 1.0 g (5.26 mmol) of O-[2-(pyrazole-1-yl)pyridine-5-yl]methylhydroxylamine in 5 mL ethanol, a solution of trichloroacetic acid (10.5 mmol) in 5 mL ethanol was added dropwise. The precipitated crystals were collected by filtering, to give 1.6 g of an objective product (yield 86.1%).
¹H NMR (DMSO 300 MHz) d4.63 (s, 2H), 6.17 (brs, 2H), 6.58 (dd, 1H), 7.74 (dd, 1H, J= 0.8, 1.7Hz), 7.83 (dd, 1H, J= 1.2 Hz), 7.93 (m, 2H),8.42 (dd, 1H), 8.62 (dd, 1H, J= 2.7 Hz)
m.p.= 127.2-127.3°C (decomposed)

| Element analysis | | | | | |
|---|---|---|---|---|---|
| C₁₁H₁₁Cl₃N₄O₃ | Calc.: | H(%) 3.14, | C(%) 37.36, | N(%) 15.85, | Cl(%) 30.08 |
| | Found: | H(%) 3.11, | C(%) 37.40, | N(%) 16.08, | Cl (%) 30.23 |

### [Example 16]

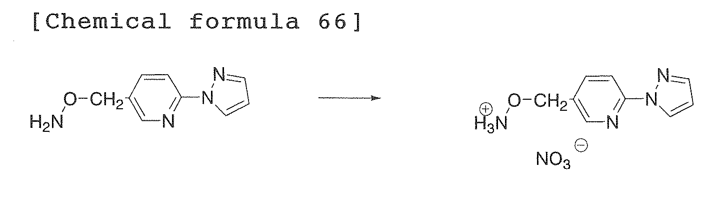

To a solution of 500 mg (2.63 mmol) of O-[2-(pyrazole-1-yl)pyridine-5-yl]methylhydroxylamine in 2.5 mL ethanol, 200 mg (3.18 mmol) of 69% nitric acid was added dropwise. Crystals that were precipitated by ice cooling were collected by filtering, to give 0.25 g of an objective O-[2-(pyrazole-1-yl)pyridine-5-yl]methylhydroxylamine nitrate (yield 37.6%).
¹H NMR (DMSO 300 MHz) d5.05 (s, 2H), 6.60 (s, 1H) 7.86 (dd, 1H, J= 0.6 Hz), 8.00(m, 2H), 8.50 (dd, 1H, J= 1.5 Hz), 8.64 (dd, 1H , J= 2.4 Hz)
m.p.= 170.5-170.6°C (decomposed)
Element analysis C₉H₁₁N₅O₄ Calc.: H(%) 4.38, C(%) 42.69, N(%) 27.66 Found: H(%) 4.28, C(%) 42.66, N(%) 27.84

### [Example 17]

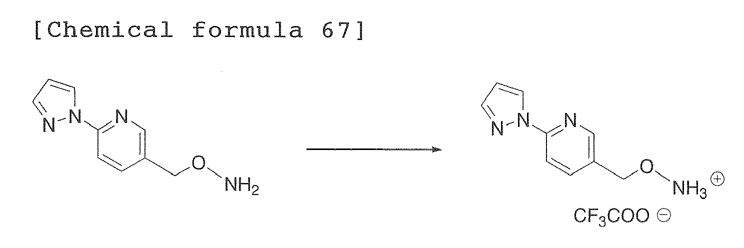

A solution of 2.0 g (10.5 mmol) of 0-[2-(pyrazole-1-yl)pyridine-5-yl]methylhydroxylamine in 12 mL toluene was added dropwise to a solution of 1 . 3g (1. 05 eq, 11.0 mmol) of TFA in mixed solvent of toluene 8 mL/IPA 1 mL. Precipitated crystals were collected by filtering, to give 2.67 g of objective O-[2-(pyrazole-1-yl)pyridine-5-yl]methylhydroxylamine trifluoroacetate (yield 83.5%).
¹H NMR (DMSO 300 MHz) d4.99 (s, 2H), 6.59 (d, 1H, 1.2= Hz), 7.84 (dd, 1H), 8.00 (m, 2H), 8.47 (dd, 1H), 8.64 (dd, 1H, J= 2.4 Hz) m.p.= 117.7°C (decomposed)

| Element analysis | | | | | |
|---|---|---|---|---|---|
| C₁₁H₁₁F₃N₄O₃ | Calc.: | H(%) 3.64, | C(%) 43.43, | N(%) 18.42, | F(%) 18.73 |
| | Found: | H(%) 3.61, | C(%) 43.48, | N(%) 18.49, | F(%) 18.89 |

### [Industrial applicability]

Through a compound of the present invention, it is possible to efficiently produce a compound shown by formula: 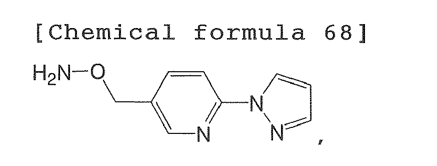 and to efficiently produce an antimicrobial agent shown by formula: 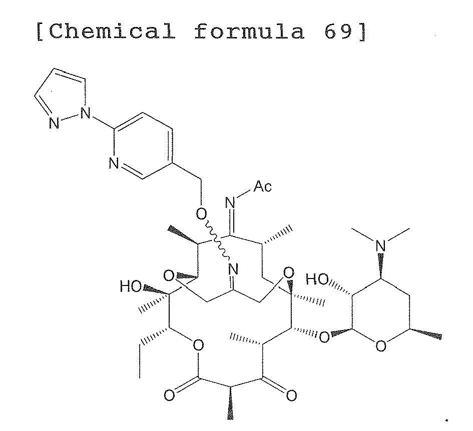 The production method of the present invention can be conducted efficiently because the number of steps is smaller than that of conventional arts. Additionally, it can industrially practiced because no reducing step is included.

## Claims

1. A compound shown by formula: 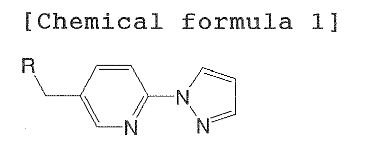 (wherein R is a protected hydroxy or a group shown by formula: 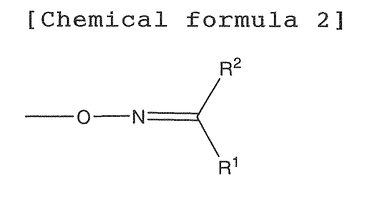 (wherein R¹ and R² each independently represent hydrogen, alkyl, optionally substituted aralkyl, optionally substituted aryl or optionally substituted heteroaryl)).

2. A method of producing compound shown by formula V 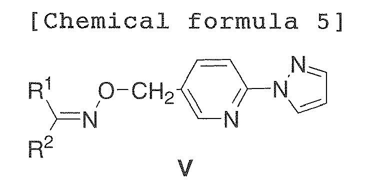 (wherein R¹ and R² each independently represent hydrogen, alkyl, optionally substituted aralkyl, optionally substituted aryl or optionally substituted heteroaryl), comprising reacting a compound shown by formula III: 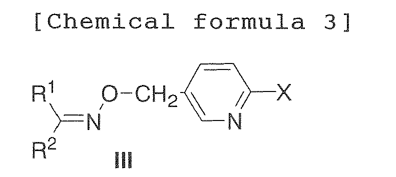 (wherein R¹ and R² are as defined above, X is halogen), with a compound shown by formula IV: 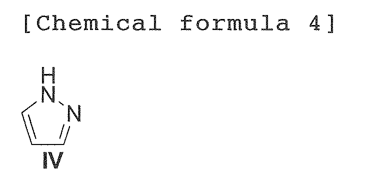 in the presence of base or acid.

3. A method of producing a compound shown by formula VI or its salt: 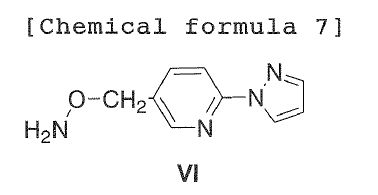 comprising hydrolyzing a compound shown by formula V: 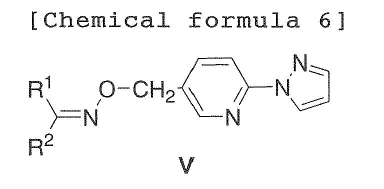 (wherein R¹ and R² each independently represent hydrogen, alkyl, optionally substituted aralkyl, optionally substituted aryl or optionally substituted heteroaryl) in the presence of acid.

4. A method of producing a compound shown by formula IX: 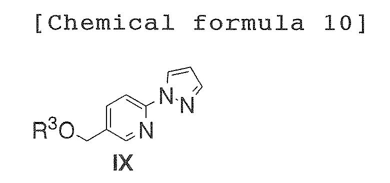 (wherein R³ represents a protective group of hydroxy) comprising reacting a compound shown by formula VIII: 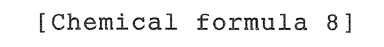 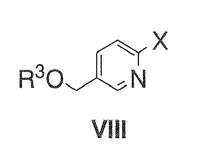 (wherein R³ is as defined above, and X is halogen), with a compound shown by formula IV : 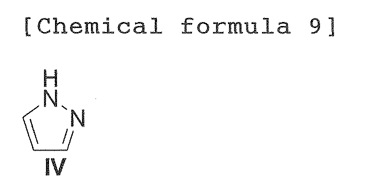 in the presence of base or acid.

5. A method of producing a compound shown by formula X: 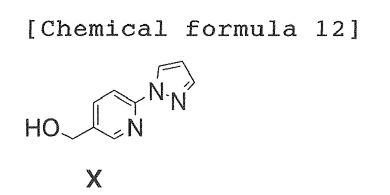 comprising hydrolyzing a compound shown by formula: 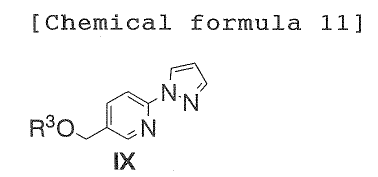 (wherein R³ represents a protective group of hydroxy) in the presence of acid.

6. A method of producing a compound shown by formula XI: 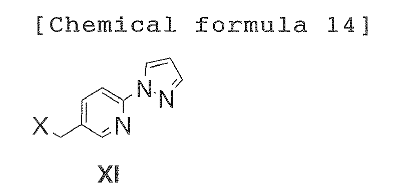 (wherein R is halogen) comprising obtaining a compound shown by formula X: 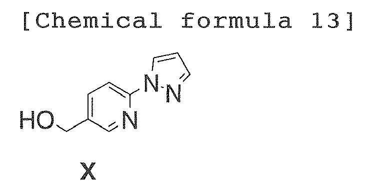 according to the method according to claim 5, and reacting the compound shown by formula X with a halogenating agent.

7. A method of producing a compound shown by formula XIII 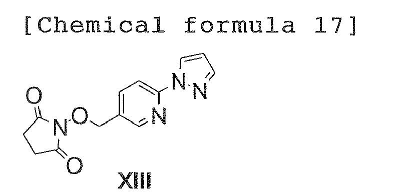 comprising obtaining a compound shown by formula XI: 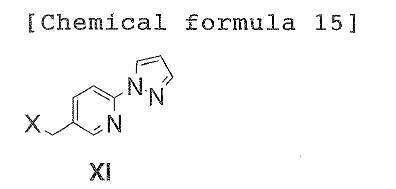 (wherein X is halogen)
according to the method according to claim 6, and reacting the obtained compound with a compound shown by formula XII:

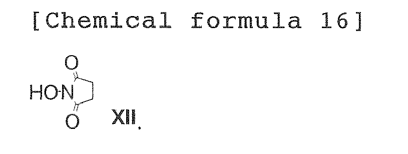

8. A method of producing a compound or its salt shown by formula VI : 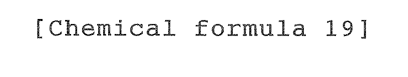 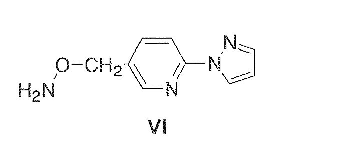 comprising obtaining a compound shown by formula XIII: 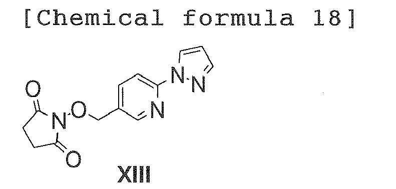 according to the method of claim 7, and hydrolyzing the obtained compound in the presence of base.

9. A method of producing a compound shown by formula: 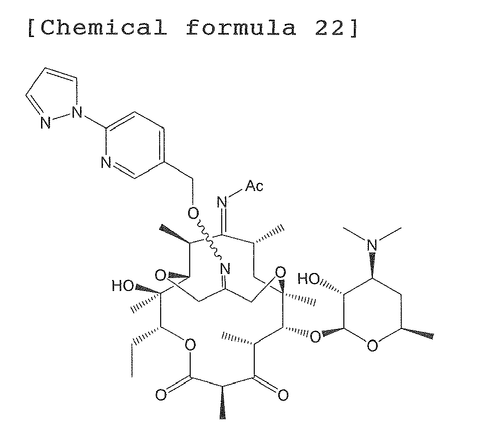 comprising obtaining a compound or its salt shown by formula VI: 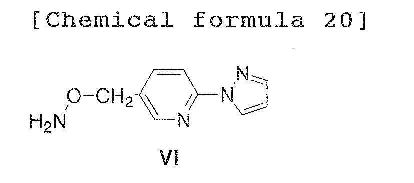 according to the method of claim 3 or claim 8, and reacting the obtained compound or its salt with a compound shown by formula: 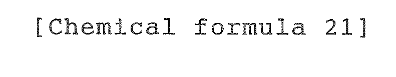 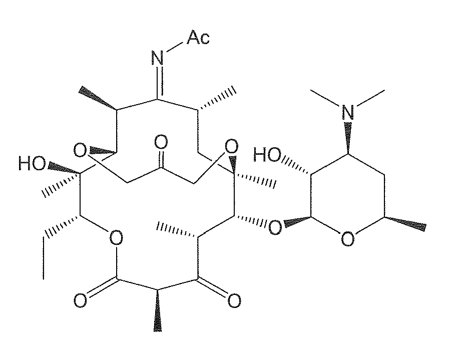 in the presence of acid or base.

10. A method of producing a compound shown by formula: 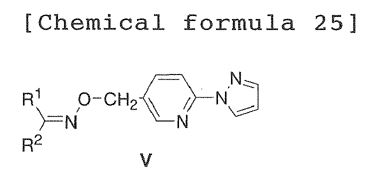 (wherein R¹ and R² each independently represent hydrogen, alkyl, optionally substituted aralkyl or optionally substituted aryl) comprising reacting a compound shown by formula: 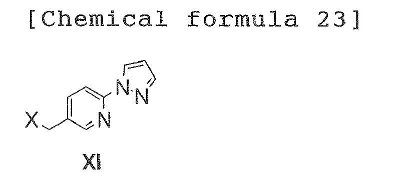 (wherein X is halogen), with a compound shown by formula: 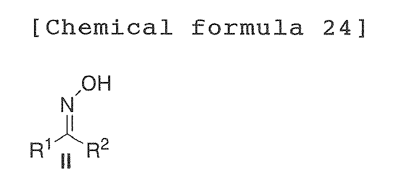 (wherein R¹ and R² are as defined above).

11. A salt of a compound shown by formula VI:

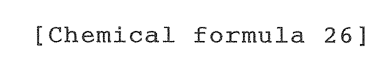

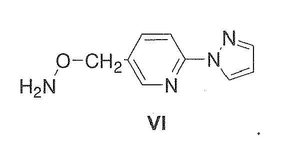

12. The salt according to claim 11 which is a hydrochloride, sulfate, trichloroacetate or trifluoroacetate.

13. A compound shown by formula: 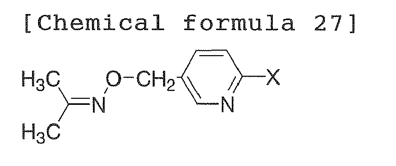 (wherein X is halogen).
